# EUROPEAN PATENT APPLICATION

(11) **EP 3 207 878 A1**
(43) Date of publication of application: **23.08.2017**
(21) Application number: 15850434.0
(22) Date of filing: 06.10.2015
(51) Int. Cl.: A61B 8/14

(54) **ULTRASOUND DIAGNOSTIC DEVICE**

(30) Priority: 15.10.2014 JP 2014210483
(71) Applicant: Hitachi, Ltd., Chiyoda-ku, Tokyo 100-8280 (JP)
(72) Inventor: HISATSU, Masanori, Mitaka-shi Tokyo 181-8622 (JP); KURIBARA, Hiroshi, Mitaka-shi Tokyo 181-8622 (JP)
(74) Representative: Strehl Schübel-Hopf & Partner
(86) International application number: PCT/JP2015/078354
(87) International publication number: WO 2016/060017

(57) **Abstract**

A plurality of transmit foci (a plurality of virtual sources) (F1-F7) are formed upon a scan plane with a two-dimensional pattern. Specifically, first transmission beams having first transmission foci and second transmission beams having second transmission foci are formed alternately in a scan direction. A plurality of first sub-images (LRI1, LRI3, LRI7) are formed by the forming of the plurality of first transmission beams, and a plurality of second sub-images (LRI2, LRI4, LRI6) are formed by the forming of the plurality of second transmission beams. At time of reception, a parallel reception technology is applied.

## Description

### TECHNICAL FIELD

The present invention relates to an ultrasound diagnostic device, and particular to an ultrasound diagnostic device that forms an ultrasonic image based on a synthetic aperture imaging method (virtual source method) using a virtual source.

### BACKGROUND

First, typical transmission and reception methods employed in an ultrasound diagnostic device will be described. Forming of a transmission beam and forming of a reception beam is repeated while transmission and reception positions (or transmission and reception directions) of the beams are changed. Specifically, during transmission, one transmission focal point is normally formed, and a transmission beam having an hourglass-like shape is formed. In that case, a good scanning direction resolution can be obtained near the transmission focal point. However, the scanning direction resolution degrades as a position is separated from the transmission focal point to the shallower side or the deeper side. During reception, reception dynamic focus technology is normally applied. Namely, the depth of a reception focal point is dynamically changed so that the reception focal point sequentially matches a plurality of reception points arranged in the depth direction. Parallel reception technology that forms a plurality of reception beams simultaneously for one transmission beam is also known. In either case, in the conventional, typical transmission and reception methods, each reception point on the scanning plane is basically associated with only one transmission beam.

The synthetic aperture imaging method using virtual sources (virtual source method) is a method of forming an ultrasonic image using synthetic transmission aperture imaging technology on the assumption that a plurality of transmission focal points are regarded as a plurality of sound sources (virtual sources) (Non-Patent Document 1, Patent Document 1, and Patent Document 2). In the virtual source method, upon delay processing of a group of reception signals, in addition to the conventionally used delay conditions for reception focusing, delay conditions for observing spherical waves emitted from each virtual source are added. If parallel reception is performed (or, if synthetic reception aperture imaging is performed) under the virtual source method, one low-resolution image (LRI) as one sub-image is obtained. By synthesizing a plurality of low-resolution images obtained by forming a plurality of transmission beams, an ultrasonic image as a high-resolution image is formed. In the virtual source method, when the plurality of low-resolution images are synthesized, a plurality of spherical wave components derived from a plurality of virtual sources are added per pixel (each reception point) after their phases are matched. The low-resolution images can be recognized regardless of whether before or after scan conversion processing.

There is also known a technique of directly forming an ultrasonic image based on a dataset obtained by forming a plurality of virtual sources without forming a plurality of sub-images.

### CITATION LIST

### PATENT LITERATURE

Patent Document 1: JP 62-47348 A
Patent Document 2: JP 10-277042 A

### NON-PATENT LITERATURE

Non-Patent Document 1: S. I. Nikolov, et al. "Practical Applications of Synthetic Aperture Imaging", IEEE IUS, 2010. (BK Medical)

### SUMMARY

### TECHNICAL PROBLEM

If, in the virtual source method, a plurality of transmission focal points (virtual sources) arranged in the scanning direction are formed at the same depth, as will be described in detail later, a low sound pressure region is generated between adjacent low-resolution images (or between adjacent virtual sources on the scanning plane). Such a low sound pressure region deteriorates the image quality of an ultrasonic image. Patent Document 2 discloses overcoming the issue of the above-described low sound pressure region by observing spherical waves (edge waves) emitted from each end of a transducer. However, it should be stated that the method is not enough to cancel the above low sound pressure region sufficiently.

An object of the present invention is to enhance the image quality of an ultrasonic image formed based on the virtual source method. Alternatively, an object of the present invention is to prevent or minimize occurrence of a low sound pressure area or low image quality area between adjacent sub-images (low-resolution images). Alternatively, an object of the present invention is to minimize occurrence of a low sound pressure area or low image quality area between adjacent virtual sources on the scanning plane.

### SOLUTION TO PROBLEM

(1) An ultrasound diagnostic device according to the present invention has a transmission unit that forms a plurality of virtual sources as a plurality of transmission focal points on a scanning plane, a sub-image forming unit that applies delay summing processing according to a virtual source method to a reception signal array obtained each time the virtual source is formed, thereby forming a sub-image, and a synthesis unit that synthesizes a plurality of sub-images corresponding to the plurality of virtual sources, thereby generating an ultrasonic image, and in this device, the plurality of virtual sources constitute a 2D virtual source array expanding in two dimensions on the scanning plane.
   With the above structure, a 2D virtual source array but not a 1D virtual source array is formed on the scanning plane which is an ultrasonic beam scanning area. The 2D virtual source array is composed of a plurality of virtual sources (that is, a plurality of transmission focal points) expanding in a 2D pattern on the scanning plane. When the depths of the plurality of virtual sources arranged in the scanning direction are aligned, a low sound pressure area is inevitably generated between individual adjacent sub-images. In contrast to this, if the plurality of virtual sources arranged in the scanning direction have at least two or more types of depths, it is possible to reduce the number of occurrences of low sound pressure areas (or make it zero), or reduce the total area of low sound pressure areas (or make it zero). Preferably, the 2D pattern for the plurality of virtual sources is determined so that a low sound pressure area is not generated in at least a main portion (central portion) in the scanning plane which is an object to be observed.
   In the above structure, a transmission beam is formed by exciting a plurality of transducer elements in transmission apertures. Preferably, the above reception signal array is composed of a plurality of element reception signals output from a plurality of transducer elements in a reception aperture. Delay summing processing according to the virtual source method is processing in which a delay time corresponding to at least the distance from a virtual source to each reception point is considered. In at least a part of the scanning plane, each reception point belonging to that part is associated with a plurality of virtual sources; that is, it is covered with a plurality of sub-images. This enables application of synthetic transmission aperture imaging processing to that reception point. However, a reception point in a part of the scanning plane may be associated with only one virtual focal point. Particularly preferably, each reception point is associated with a plurality of virtual sources on substantially the entire scanning plane. In terms of image quality improvement, it is preferable that as many reception points as possible are associated with as many virtual sources as possible. Preferably, during reception, the parallel reception technology is applied; namely, a plurality of reception beams are formed in parallel after one transmission beam is formed. The above sub-image is a concept representing a reception data array formed for each virtual source. Preferably, each data point constituting the sub-image is RF data obtained before it undergoes detection processing. The above 2D virtual source array may be formed on at least one plane (scanning plane) in a 3D data input space.
   Preferably, the virtual source array includes a plurality of first virtual sources set at a first depth and a plurality of second virtual sources set at a second depth that differs from the first depth. Preferably, the plurality of first virtual sources and the plurality of second virtual sources are formed alternately in the scanning direction. Because, with such a structure, two types of sub-images having different shapes can be obtained alternately, it becomes easier to exclude a low sound pressure area between individual adjacent sub-images.
   Preferably, the transmission unit has a function of transmitting ultrasonic waves from a first transmission aperture having a first aperture size, thereby forming a first transmission beam having a first transmission focal point set at the first depth, and a function of transmitting ultrasonic waves from a second transmission aperture having a second aperture size that differs from the first aperture size, thereby forming a second transmission beam having a second transmission focal point set at the second depth, the sub-image forming unit has a function of forming a first reception beam data array based on a first reception signal array obtained by receiving reflected waves after the first transmission beam is formed, and a function of forming a second reception beam data array based on a second reception signal array obtained by receiving reflected waves after the second transmission beam is formed, and in this device, a first sub-image is formed based on the first reception beam data, and a second sub-image is formed based on the second reception beam data.
   Preferably, the first transmission beam has a first beam shape composed of a small inversed triangle area expanding from the first transmission focal point to the near side and a large triangle area expanding from the first transmission focal point to the far side, and the second transmission beam has a second beam pattern composed of a large inversed triangle area expanding from the second transmission focal point to the near side and a small triangle area expanding from the second transmission focal point to the far side. Here, the near side is a side shallower than the transmission focal point, and the far side is a side deeper than the transmission focal point.
   Preferably, the sub-image forming unit includes a delay summing processing unit that applies delay summing processing according to the virtual source method to a reception signal array obtained each time the virtual source is formed, thereby forming a reception beam data array, and a weighting unit that applies a weight distribution to the reception beam data array. Weighting processing includes, for example, processing for suppressing each data point belonging to the outside of the sub-images and weighting processing for overcoming unbalanced sound pressure in a sub-image. It is preferable to perform weighting processing so as not to generate the uneven quality in a final ultrasonic image as much as possible. Preferably, the weighting unit provides a weight for invalidation to a portion of the reception beam data array, the portion deviated from a transmission beam area. With this structure, it is possible to form sub-images including a group of data to be invalidated for the time being and remove or suppress a group of unnecessary data later at the same time as general weighting.
   Preferably, the transmission unit includes a function of transmitting ultrasonic waves from a first transmission aperture having a first aperture size, thereby forming a first transmission beam having a first transmission focal point set at the first depth, and a function of transmitting ultrasonic waves from a second transmission aperture having a second aperture size that differs from the first aperture size, thereby forming a second transmission beam having a second transmission focal point set at a second depth that differs from the first depth, the sub-image forming unit includes a function of forming a first reception beam data array based on a first reception signal array obtained by receiving reflected waves after the first transmission beam is formed, and a function of forming a second reception beam data array based on a second reception signal array obtained by receiving reflected waves after the second transmission beam is formed, and the weighting unit includes a function of applying a first weight distribution to the first reception beam data, thereby forming a first sub-image, and a function of applying a second weight distribution to the second reception beam data, thereby forming a second sub-image. Because forming conditions of the first sub-image and forming conditions of the second sub-image differ, it is preferable to prepare weight distributions respectively corresponding to the first and second sub-images, and apply suitable weight distributions to them. Further, a weight distribution may be prepared individually for each of the sub-images at different positions in the scanning direction.
(2) The ultrasound diagnostic device according to the present invention includes a transmission unit that forms a plurality of virtual sources as a plurality of transmission focal points on a scanning plane, a storage unit that stores, as a dataset, a plurality of reception signal arrays obtained by forming the plurality of virtual sources, and a synthesis unit that applies delay summing processing according to the virtual source method to the dataset, thereby generating an ultrasonic image, and in this device, the plurality of virtual sources constitute a 2D virtual source array expanding in two dimensions on the scanning plane.
   In the above structure, a dataset is obtained by formation of the plurality of virtual sources as the plurality of transmission focal points, and by applying delay summing processing to the dataset based on the virtual source method, an ultrasonic image is formed. In this processing, the ultrasonic image is formed directly from the dataset without forming a plurality of sub-images. In such a case, if the plurality of virtual sources are formed at the same depth, a low sound pressure region is inevitably generated between the virtual sources on the scanning plane. Because, with the above structure, the plurality of virtual sources expand in two dimensions, it is possible to avoid or reduce occurrence of the low sound pressure regions.
   Preferably, the virtual source array includes a plurality of first virtual sources set at a first depth and a plurality of second virtual sources set at a second depth that differs from the first depth. Preferably, the plurality of first virtual sources and the plurality of second virtual sources are formed alternately in the scanning direction.

### BRIEF DESCRIPTION OF DRAWINGS

[FIG. 1] FIG. 1 is a diagram showing a general transmission and reception method in an ultrasound diagnostic device.
[FIG. 2] FIG. 2 is a diagram showing a synthetic transmission aperture imaging method (virtual source method) based on virtual sources.
[FIG. 3] FIG. 3 is a diagram showing generation of a plurality of sub-images (low-resolution images).
[FIG. 4] FIG. 4 is a block diagram showing an embodiment of an ultrasound diagnostic device according to the present invention.
[FIG. 5] FIG. 5 is a diagram showing a first example of a first transmission and reception beam set.
[FIG. 6] FIG. 6 is a diagram showing a first example of a second transmission and reception beam set.
[FIG. 7] FIG. 7 is a diagram showing a first example of a 2D virtual source array.
[FIG. 8] FIG. 8 is a diagram showing a second example of a 2D virtual source array.
[FIG. 9] FIG. 9 is a conceptual diagram for illustrating a first example of processing at a reception unit.
[FIG. 10] FIG. 10 is a conceptual diagram for illustrating a second example of processing at the reception unit.
[FIG. 11] FIG. 11 is a diagram showing an electronic sector scanning method to which the virtual source method is applied.
[FIG. 12] FIG. 12 a diagram showing a second example of the first transmission and reception beam set.
[FIG. 13] FIG. 13 a diagram showing a second example of the second transmission and reception beam set.
[FIG. 14] FIG. 14 is a diagram showing another structure example of the reception unit.

### DESCRIPTION OF EMBODIMENTS

Hereinafter, preferred embodiments of the present invention will be described by reference to the drawings.

### (1) General Transmission and Reception Method

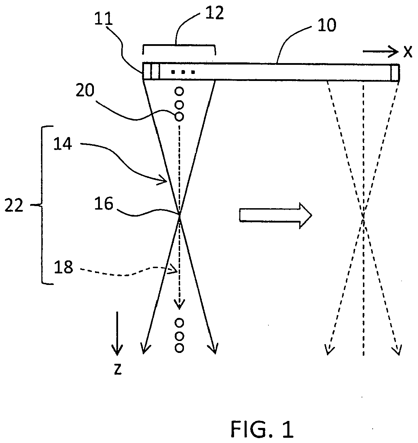

First, a general transmission and reception method will be described with reference to FIG. 1. An array transducer 10 is composed of a plurality of transducer elements 11 arranged linearly along the x direction in FIG. 1. In FIG. 1, the x direction is a scanning direction (element array direction), and the z direction is a depth direction (beam direction). A transmission aperture 12 is set on the array transducer 10, and by exciting the plurality of transducer elements belonging to it with a certain delay relationship, a transmission beam 14 is formed. The transmission beam 14 has a transmission focal point 16 formed at a set depth. In the transmission beam 14, the shallower side (upper side) and the deeper side (lower side) in relation to the transmission focal point become gradually wider as they go further away from the transmission focal point. In each drawing, the transmission beam is depicted schematically.

After the transmission beam is formed, reflected waves are received, thereby obtaining an element reception signal array. During the process, the reception dynamic focus technology is applied, and with this technology, reception beams are formed electronically. Reference numeral 18 represents a piece of reception beam data corresponding to a reception beam. The reception beam data 18 is obtained by applying delay summing processing while dynamically changing delay conditions for a plurality of element reception signals output from a plurality of transducer elements in a reception aperture. Delay summing processing is also referred to as phasing alignment processing which is known processing. The reception beam data 18 is composed of a plurality of pieces of reception data (echo data) 20 corresponding to a plurality of reception points (sample points) arranged in the depth direction. There are cases where the transmission aperture and the reception aperture are identical, and cases where they differ.

Reference numeral 22 indicates a transmission and reception beam set composed of the transmission beam 14 and the reception beam (in FIG. 1, the reception beam data 18 corresponding to the reception beam is shown). By forming the transmission and reception beam set 22 at each position in the scanning direction, a plurality of pieces of reception beam data 18 equivalent to one scanning plane; that is, one reception frame, can be obtained. In some cases, there is applied the parallel reception technology that forms, for one transmission beam 14, a plurality of reception beams arranged in the scanning direction.

### (2) Synthetic Aperture Imaging Method Using Virtual Source (Virtual Source Method)

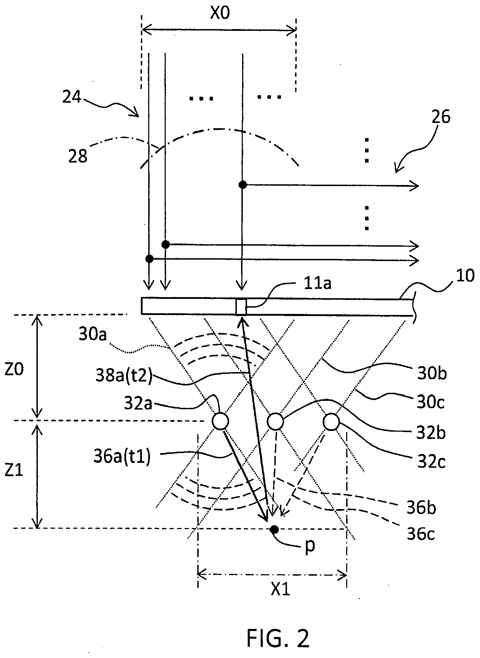

FIG. 2 shows an example of the virtual source method. In the shown example, a transmission signal line array 24 is connected to the array transducer 10, and a reception signal line array 26 is drawn out from the middle of the signal line array 24. A transmission aperture X0 is set for the array transducer 10, and the plurality of transducer elements belonging to it are provided with a plurality of transmission signals having a delay relationship indicated by a delay curve 28. Then, a transmission beam 30a formed. The transmission beam 30a has a transmission focal point 32a. Likewise, a transmission beam 30b, a transmission beam 30c, ...are formed while the transmission position is changed in the scanning direction. Those transmission beams have a transmission focal point 32b, a transmission focal point 32c .... In FIG. 2, they have the same depth, that is, Z0. Although not shown in FIG. 2, in this example, a plurality of reception beams are formed for each transmission beam so as to cover the whole or the main part of the transmission beam area by applying the parallel reception technology.

A reception point p will now be examined. In the shown example, the reception point p is covered with the three transmission beams 30a, 30b, and 30c. In other words, the reception point p is associated with the three transmission beams 30a, 30b, and 30c. Each transmission focal point 32a, 32b, and 32c can be regarded as a virtual source (hereinafter, depending on circumstances, "transmission focal point" will be referred to as "virtual source"). That is, it is possible to recognize, on the near side and the far side of each virtual focal point, spherical waves having each virtual source as an origin. FIG. 2 shows spherical waves coming from the virtual source 32a. Three spherical wave components 36a, 36b, and 36c respectively derived from the three virtual sources 32a, 32b, and 32c arrive at the reception point p. By matching phases of those spherical wave components and synthesizing the results, it is possible to observe a large amplitude in the reception point p. That is, for example, in reception processing after the transmission beam 30a is formed, delay summing processing is performed in consideration of the delay conditions corresponding to the distance between the virtual source 32a and the reception point p in addition to the delay conditions for realizing normal reception dynamic focus at the reception point p. In FIG. 2, the distance from the reception point p to a particular transducer element 11a in the reception aperture is indicated by reference numeral 38a, and a propagation time for that is t2. In reception dynamic focus, a delay time is provided to an element reception signal from the transducer element 11 a based on the propagation time t2. In synthetic transmission aperture imaging, a delay time is provided to that element reception signal based on a propagation time t1 of a spherical wave. Similar reception processing is also performed in reception processing after the transmission beams 30b and 30c are formed. Non-Patent Document 1 provides a detailed description of a delay time for realizing synthetic transmission aperture imaging. As the number of transmission beams covering the reception point p becomes greater (as the number of synthetic transmission apertures becomes greater), a larger amplitude can be obtained at the reception point; that is, the image quality of an ultrasonic image can be enhanced. Conversely, because, with the virtual source method, the image quality of an ultrasonic image can be enhanced, it is possible to reduce the number of transmission beams accordingly and improve the frame rate.

In the virtual source method, at the reception point p, a virtual source range (that is, a virtual source aperture) used in synthetic transmission aperture imaging is equivalent to the scanning direction width of the transmission beam at the depth where the reception point p exists. Specifically, the further the reception point p is separated from the virtual source 32b, the larger the virtual source aperture X1 becomes in proportion to the distance Z1 in the depth direction. That is, the virtual source method always has the same f^{#} (= Z1/X1), regardless of the depth of the reception point. This f^{#} is determined by an actual transmission aperture X0 and transmission focal point distance Z0, and f^{#} = Z0/X0.

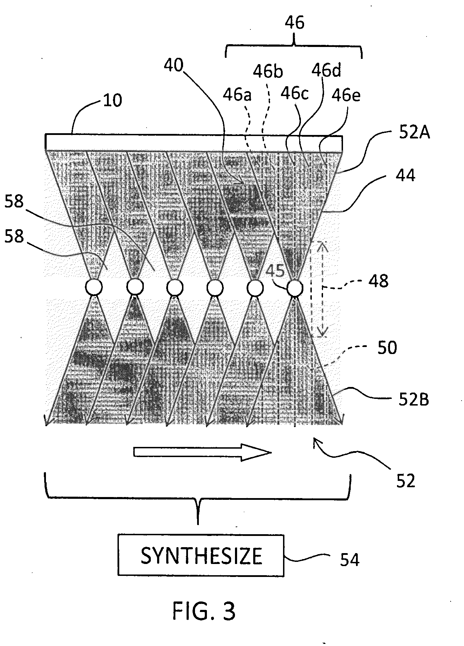

FIG. 3 shows a plurality of sub-images 40. One sub-image 40 corresponds to one transmission and reception beam set. In the shown example, a reception beam array 46 is formed for one transmission beam 44 so as to cover it. The reception beam array 46 is composed of a plurality of reception beams 46a to 46e arranged in the scanning direction. However, in the reception beam array 46, a portion deviated from an area of the transmission beam 44 is considered to be invalid because the sensitivity suitable for imaging cannot be obtained from it. For example, an interval 48 near the transmission focal point 45 in the reception beam 46d is an invalid portion. The other portions of the reception beam 46d are valid portions. Invalidation processing of data in the invalid portion can be performed, for example, when the reception beam data is formed or when weighting processing is performed later, as will be described later.

The reception beam array 46 is a 2D reception data array; that is, it constitutes a sub-image 52. Because the sub-image 52 is an image obtained before synthetic transmission aperture imaging processing, it is a low-resolution image. The sub-image 52 is composed of an inversed triangle portion 52A present on the near side of the transmission focal point 45 and a triangle portion 52B present on the far side of the transmission focal point 45. In the example shown in FIG. 3, a plurality of sub-images constituting an ultrasonic image have the same shape.

As shown in FIG. 3, a low sound pressure region 58 is generated as a gap between individual adjacent sub-images. In FIG. 3, each low sound pressure region 58 is represented as a figure having a diamond shape. Each low sound pressure region 58 is a region deviated from the area of the transmission beam 44, and is an area that is not suitable for imaging. Therefore, in processing of the reception beam data, the invalidation processing described above is applied. If a plurality of sub-images 52 are synthesized to form an ultrasonic image, a plurality of low image quality regions corresponding to the plurality of low sound pressure regions 58 are generated. As such, if the plurality of virtual sources are uniformly set at the same depth, it is difficult to enhance the image quality of an ultrasonic image.

### (3) Structure of Ultrasound Diagnostic Device

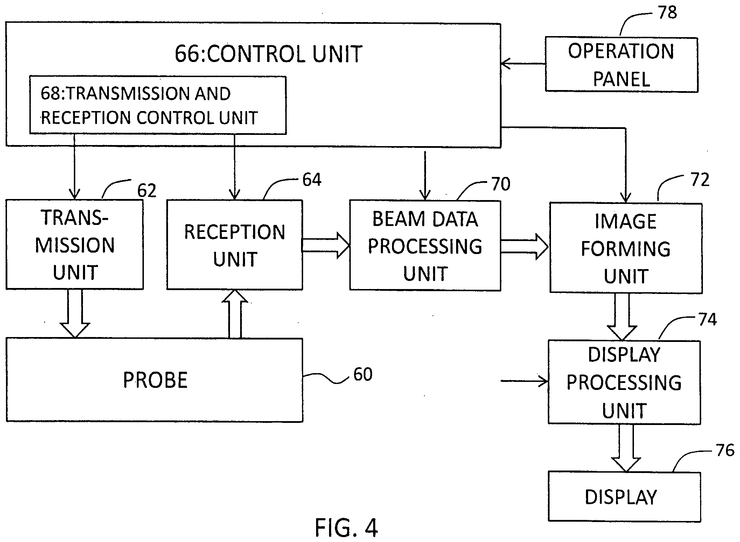

FIG. 4 shows a preferred embodiment of an ultrasound diagnostic device according to the present invention. This ultrasound diagnostic device is located in medical organizations and performs ultrasonic diagnosis of the human body. This ultrasound diagnostic device has a function of forming an ultrasonic image according to the virtual source method. The ultrasonic image is a B-mode tomography image, for example. As will be described in detail later, in the present embodiment, a plurality of virtual sources are arranged in two dimensions so as not to generate the low sound pressure regions described above.

In FIG. 4, a probe 60 is a transducer that transmits and receives ultrasonic waves while it is in contact with a living body. The probe 60 has an array transducer. The array transducer forms an ultrasonic beam, and that ultrasonic beam is electronically scanned. In the present embodiment, an electronic linear scanning method is used as an electronic scanning method. Other electronic scanning methods include an electronic sector scanning method, for example. An electronic convex scanning method is known as an embodiment of the electronic linear scanning method.

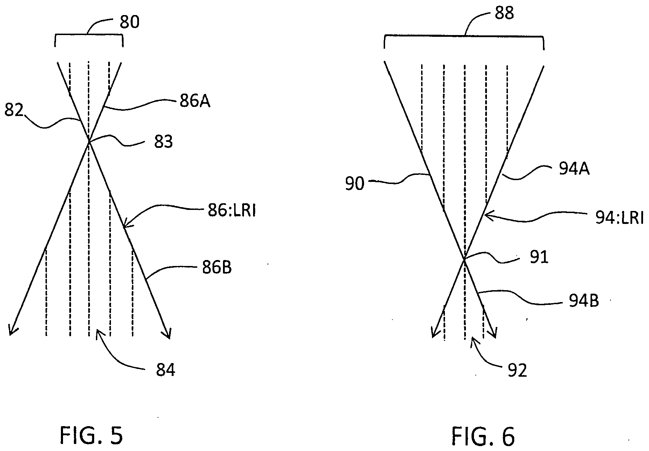

A transmission unit 62 is a transmission beam former configured as an electronic circuit. The transmission unit 62 provides the array transducer with a plurality of transmission signals having a predetermined delay relationship. In doing so, a transmission beam is formed. A transmission beam is formed repeatedly, while its transmission position is changed in the scanning direction. In the present embodiment, a first transmission beam having a first transmission focal point at a first depth and a second transmission beam having a second transmission focal point at a second depth are formed alternately. The first depth is set, for example, at a shallow position, and the second depth is set, for example, at a position that is deeper than the first depth. Upon forming of the first transmission beam, for example, a small first transmission aperture is set, and upon forming of the second transmission beam, for example, a second transmission aperture that is larger than the first transmission aperture is set. The shape of the first transmission beam and the shape of the second transmission beam differ from each other. As described above, a 2D pattern of a plurality of transmission focal points (that is, a plurality of virtual sources) is formed on the beam scanning plane. In this regard, a detailed description will be provided later with reference to FIG. 5 to FIG. 8.

A reception unit 64 is a reception beam former configured as an electronic circuit. Specifically, the reception unit 64 has a parallel reception function of forming a plurality of reception beams (a plurality of pieces of reception beam data) for one transmission beam. Upon forming of the individual reception beams, the reception dynamic focus technology is applied. Specifically, the reception unit has a delay circuit and an adder circuit that respectively perform delay processing and summing processing. In addition, the reception unit has an A/D converter or the like. The delay summing processing described above is composed of delay processing and summing processing. In the delay circuit, delay processing is performed on a plurality of element reception signals taken from the reception aperture. In that case, a delay time includes a delay time for reception dynamic focus and a delay time for spherical wave components from the virtual sources (transmission focal points). Namely, delay processing according to the virtual source method is performed. The plurality of element reception signals subjected to delay processing are added in the adder circuit, and, with this process, reception beam data is obtained. In reality, a plurality of pieces of reception beam data are simultaneously obtained for one transmission beam by time-division processing. Those pieces of reception beam data constitute a sub-image. Each reception beam data is composed of a plurality of pieces of reception data corresponding to a plurality of reception points, and each piece of reception data is equivalent to an RF signal.

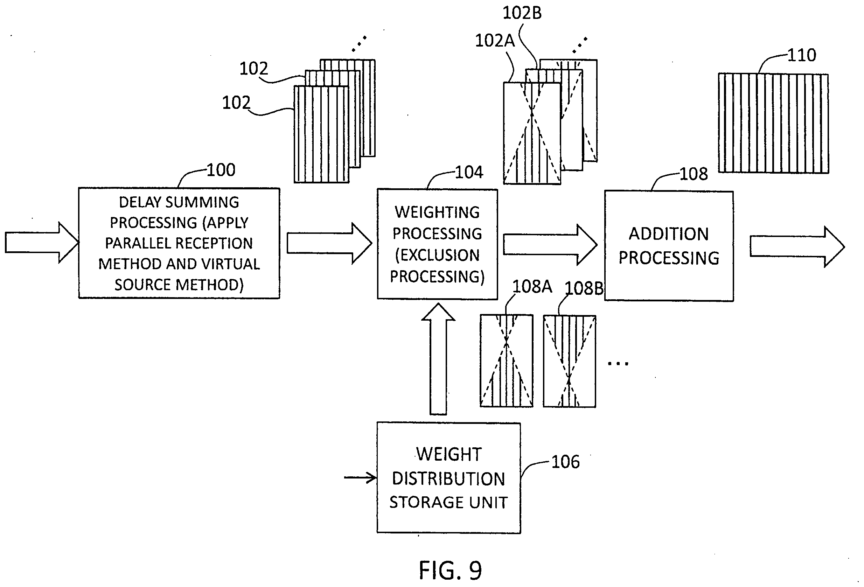

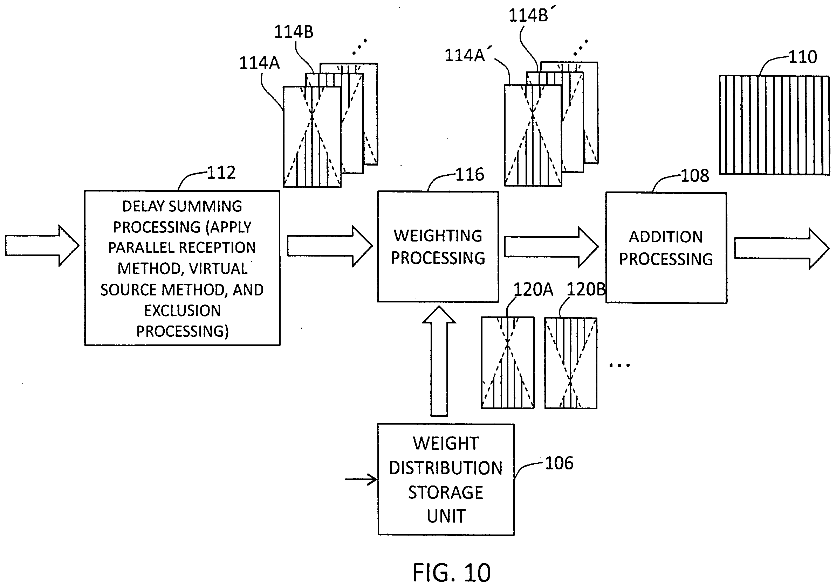

In the present embodiment, the reception unit 64 has a synthetic circuit that synthesizes the plurality of sub-images spatially and forms an ultrasonic image. The ultrasonic image is a data array before scan conversion. By reception processing subsequent to the first transmission beam, a first sub-image having a shape according to the shape of the first transmission beam is generated. By reception processing subsequent to the second transmission beam, a second sub-image having a shape according to the shape of the second transmission beam is generated. Due to scanning of the ultrasonic beams, the first sub-image and the second sub-image are obtained alternately. The synthetic circuit adds those sub-images sequentially, and forms an ultrasonic image in the end. Upon sequential synthesizing of the sub-images, the below-described weighting processing is performed. Functions of the reception unit 64 will be described later with reference to FIG. 9 and FIG. 10.

A control unit 66 is composed of a CPU and an operation program, and its transmission and reception control function is indicated as a block (transmission and reception control unit) 68 in FIG. 4. The transmission and reception control unit 68 controls the transmission unit 62 and the reception unit 64, in order to realize the virtual source method. A group of parameters that determine a 2D virtual source array are set by the transmission and reception control unit 68.

A beam data processing unit 70 is provided with circuits, such as a detector circuit and a logarithmic compressor circuit. Each reception beam data is processed by them in a stepwise manner. The reception beam data subjected to their processing are sent to an image forming unit 72. The image forming unit 72 is composed of a digital scan converter as an electronic circuit. It has functions of, for example, converting a data array according to a transmission and reception coordinate system to a data array according to a display coordinate system, interpolating the data, and adjusting the frame rate. A display frame data is generated in the image forming unit 72 and sent to a display 76 via a display processing unit 74. The display 76 displays the display frame data. For example, a B-mode tomographic image is displayed on the screen. Because, in the present embodiment, low sound pressure regions that are generated during synthetic transmission aperture imaging are prevented from occurring, it is possible to enhance the image quality of the B-mode tomographic image. An operation panel 78 is an input device which has, for example, a track ball and a keyboard. The control unit 66 executes operation control of each component shown in FIG. 4.

### (4) 2D Array Virtual Source Method

FIG. 5 and FIG. 6 schematically show a first transmission and reception beam set and a second transmission and reception beam set that are formed alternately in the ultrasound diagnostic device according to the present embodiment.

The first transmission and reception beam set shown in FIG. 5 is composed of a transmission beam 82 having a first transmission focal point 83 present at a first depth, and a reception beam array 84 provided so as to cover it. Reference numeral 80 indicates a first transmission aperture. The reception beam array 84 is composed of a plurality of reception beams arranged in the scanning direction, and, except for the reception beam in the center, each reception beam has an invalid portion which is a portion generated on the outside of the transmission beam area. The reception beam array is equivalent to a reception beam data array which is equivalent to a sub-image 86.

The second transmission and reception beam set shown in FIG. 6 is composed of a transmission beam 90 having a second transmission focal point 91 present at a second depth that is deeper than the first depth, and a reception beam array 92 provided so as to cover it. Reference numeral 88 indicates a second transmission aperture. That second transmission aperture 88 is larger than the first transmission aperture 80. The reception beam array 92 is composed of a plurality of reception beams arranged in the scanning direction, and, except for the reception beam in the center, each reception beam has an invalid portion which is a portion generated on the outside of the transmission beam area. The reception beam array is equivalent to a reception beam data array which is equivalent to a sub-image 94.

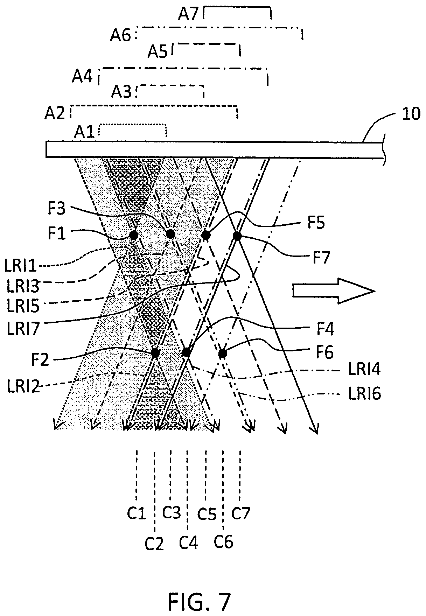

FIG. 7 shows a case where the first transmission and reception beam set and the second transmission and reception beam set are formed alternately in a repeated manner while their transmission and reception positions are changed in the scanning direction. First, a transmission aperture (first transmission aperture) A1 is set using a position C1 as the center, and a transmission beam (first transmission beam) having a transmission focal point (first transmission focal point) F1 is formed. Then, a plurality of pieces of reception beam data are obtained by delay summing processing according to the virtual source method and the parallel reception technology. They constitute a sub-image (first sub-image) LRI1. The sub-image LRI1 is composed of a small inversed triangle area present on the near side of the transmission focal point F1 and a large triangle area present on the far side of the transmission focal point F1.

Next, a transmission aperture (second transmission aperture) A2 is set using a position C2 as the center, and a transmission beam (second transmission beam) having a transmission focal point (second transmission focal point) F2 is formed. Then, a plurality of pieces of reception beam data are obtained by delay summing processing according to the virtual source method and the parallel reception technology. They constitute a sub-image LRI2. The sub-image LRI2 is composed of a large inversed triangle area present on the near side of the transmission focal point F2 and a small triangle area present on the far side of the transmission focal point F2.

After that, similarly to the above, forming of the first sub-image and forming of the second sub-image is repeated alternately. FIG. 7 shows seven sub-images LRI1 to LRI7. Each reception point belonging to a main portion (middle portion) of the scanning plane is associated with two or more transmission beams; that is, that reception point is covered with two or more sub-images. Within the scanning plane, there are also some reception points, each associated with only one transmission beam. In reality, each of a number of reception points is covered with a number of sub-images. C1 to C7 indicate center positions of the individual sub-images. F1 to F7 indicate virtual sources (transmission focal points) in the individual sub-images. A1 to A7 indicate individual transmission apertures.

When forming of the first sub-image and forming of the second sub-image are repeated alternately as described above, a 2D virtual source array having a 2D zigzag pattern is formed as a result. In that array, the depth of a certain virtual source differs from the depth of virtual sources on both sides of it. As shown in FIG. 7, the plurality of sub-images are overlapped partly in a repeated manner, thereby preventing occurrence of a plurality of low sound pressure regions. Namely, both side areas of the virtual source of the first sub-image are covered with two second sub-images present on both sides of that first sub-image. By reducing a virtual source pitch in the scanning direction, it is possible to increase the number of sub-images covering each reception point. Therefore, it is preferable to reduce the virtual source pitch, as long as a requested frame rate is satisfied. In order to prevent occurrence of a low echo portion (lacked image) in an ultrasonic image, it is preferable to determine the 2D virtual source array, the shapes of the transmission beams, and the like, so that each reception point is covered with at least one sub-area in a main area which is an object to be observed.

In order to make the scanning direction resolution uniform between the first sub-image and the second sub-image, it is preferable to make F^{#} (that is, transmission focal point distance/transmission aperture width) constant in forming two transmission beams to form two sub-images.

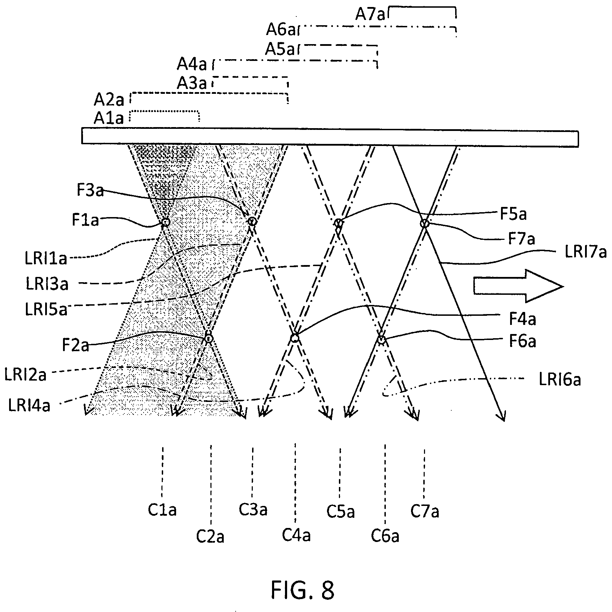

FIG. 8 shows a second example of the 2D virtual source array. In this second example, the virtual source pitch is extended to the maximum limit in the scanning direction. In this second example, the first transmission and reception beam set and the second transmission and reception beam set are also formed alternately in a repeated manner while their transmission and reception positions are changed in the scanning direction.

First, a transmission aperture (first transmission aperture) A 1 a is set using a position C 1 a as the center, and a transmission beam (first transmission beam) having a transmission focal point (first transmission focal point) F1a is formed. Then, a plurality of pieces of reception beam data are obtained by delay summing processing according to the virtual source method and the parallel reception technology. They constitute a sub-image (first sub-image) LRI1a. Next, a transmission aperture (second transmission aperture) A2a is set using a position C2a as the center, and a transmission beam (second transmission beam) having a transmission focal point (second transmission focal point) F2a is formed. Then, a plurality of pieces of reception beam data are obtained by delay summing processing according to the virtual source method and the parallel reception technology. They constitute a sub-image (first sub-image) LRI2a. In this second example, the transmission aperture A2a, the transmission focal point F2a, and the like are set such that the rear edge of the transmission beam having the transmission focal point F2a (edge on the rear side in the scanning direction) slightly overlaps with the front edge of the transmission beam having the transmission focal point F1a (edge on the near side in the scanning direction) without a gap.

Thereafter, the third and the following sub-images LRI3a to LRI7a are sequentially formed in a similar manner. In that case, the position and the shape of a sub-image to be formed next is also determined such that the next sub-image slightly overlaps with the last formed sub-image partly. C1a to C7a indicate center positions of the individual sub-images. F1a to F7a indicate virtual sources (transmission focal points) in the individual sub-images. A1a to A7a indicate individual transmission apertures.

Although, according to this second example, the number of synthetic transmission apertures is reduced (becomes one) at many reception points, it is possible to prevent lacked image in an ultrasonic image and even increase the frame rate significantly. Preferably, the 2D array including the virtual source pitch can be switched in accordance with the purpose of diagnosis or the like.

### (5) Structure of Reception Unit

FIG. 9 shows a first example of processing details in the reception unit. Each block can be composed of a hardware circuit. In a block 100, delay summing processing according to the virtual source method is performed. During that processing, in the present embodiment, the parallel reception technology is applied. Specifically, in the block 100, delay processing of an element reception signal array and summing processing of the element reception signal array subjected to delay processing is performed. By performing delay summing processing on the element reception signal array, a reception beam data array 102 is formed. Specifically, a first reception beam data array corresponding to the first transmission beam and a second reception beam data array corresponding to the second transmission beam are generated alternately. The first reception beam data array and the second reception beam data array constitute a first sub-image and a second sub-image, respectively.

A weight distribution storage unit 106 is composed of a memory. The weight distribution storage unit 106 has at least a first weight distribution 108A applied to the first reception beam data array and a second weight distribution 108B applied to the second reception beam data array. Because, in reality, transmission and reception conditions differ depending on transmission and reception positions, in the present embodiment, a weight distribution is prepared per transmission beam; that is, per reception beam data array. In a block 104, each reception beam data array is subjected to application of a corresponding weight distribution (that is, subjected to weighting processing), and with this processing, a first reception beam data array 102A subjected to weighting processing and a second reception beam data array 102B subjected to weighting processing are generated alternately. In a block 108, a plurality of reception beam data arrays subjected to weighting processing are synthesized (subjected to addition processing) to generate an ultrasonic image 110. This process is equivalent to synthetic transmission aperture imaging. In reality, the ultrasonic image 110 is composed of the plurality of pieces of reception beam data arranged in the scanning direction. Upon synthetic transmission aperture imaging, inter-line interpolation processing, inter-frame interpolation processing, and the like may be applied. The ultrasonic image 110 is sent to a beam data processing circuit which is at a later stage.

In the first example shown in FIG. 9, in the block 104, exclusion processing for invalidating data deviated from the transmission beam area is performed. That is, a point deviated from the transmission beam area is multiplied by a weight of 0. As such, by performing invalidation processing on the outside of the transmission beam area together with weighting on the inside of the transmission beam area, it is possible to reduce the amount of computation and simplify a hardware configuration.

In contrast to this, in a second example shown in FIG. 10, invalidation processing is performed on data deviated from the transmission beam area at the stage of a block 112 where delay summing processing is performed. Namely, because, in delay summing processing, delay processing is performed according to the virtual source method and the parallel reception technology, and then summing processing is performed, data deviated from the transmission area are excluded at either stage during the processing. For example, gate processing for extracting only data in the transmission area may be performed. With this processing, first and second reception beam data 114A and 114B subjected to invalidation processing are generated. In a block 116, the first and second reception beam data 114A and 114B are respectively multiplied by first and second weight distributions 120A and 120B (weighting processing). During that processing, in this second example, only weighting processing in the transmission beam area is performed. In reality, it is preferable to prepare a dedicated weight distribution for each reception beam data set. First and second reception beam data 114A' and 114B' subjected to weighting processing are sent to the block 108 and undergo addition processing there to generate the ultrasonic image 110.

### (6) Other Embodiments

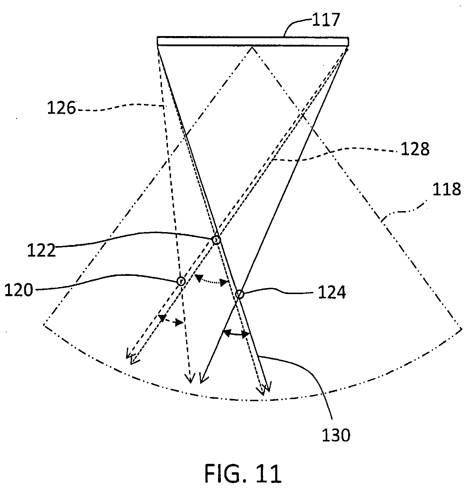

FIG. 11 shows an electronic sector scanning method based on the virtual source method. Normally, an entire array transducer 117 constitutes a transmission aperture and a reception aperture. In the electronic sector scanning, a first transmission beam and a second transmission beam are also formed alternately in orientation directions. In the shown example, a first transmission beam 126 having a first transmission focal point 120, a second transmission beam 128 having a second transmission focal point 122, and a first transmission beam 130 having a first transmission focal point 124 are formed sequentially. On a scanning plane 118, a plurality of transmission focal points; that is, a plurality of virtual sources form a 2D zigzag pattern, and in this regard, this embodiment is identical to the embodiments shown in FIG. 7 and FIG. 8. When a plurality of sub-images are formed, this embodiment also ensures prevention of occurrence of low sound pressure regions between adjacent sub-images.

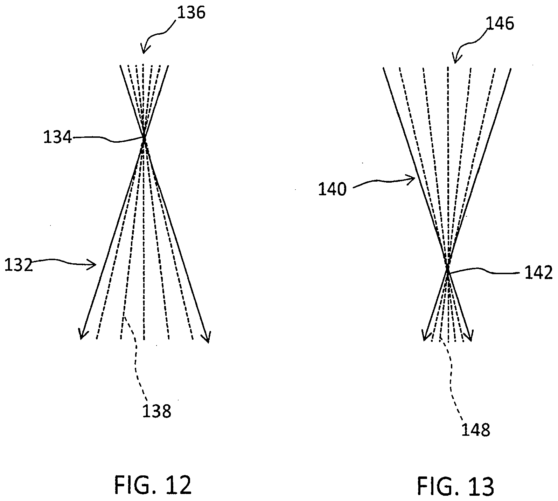

In the electronic sector scanning method, reception beam arrays, such as those shown in FIG. 12 and FIG. 13, may be formed. FIG. 13 shows a first reception beam array 146 formed in association with the above first transmission beam 140. The first reception beam array 146 is composed of a plurality of reception beams 148 crossing with each other at a transmission focal point 142. FIG. 12 shows a second reception beam array 1.36 formed in association with the above second transmission beam 132. The second reception beam array 136 is composed of a plurality of reception beams 138 crossing with each other at a transmission focal point 134. With such a structure, reception beam data do not deviate from a transmission beam area, and therefore, invalidation processing for that is unnecessary.

If, in the virtual source method, a plurality of virtual sources are set at a uniform depth as described above, low sound pressure regions are generated on both sides of each virtual source. However, it is possible to prevent occurrence of such low sound pressure regions with the 2D virtual source array described above. Because, in the parallel reception technology, the sensitivity and the resolution differ depending on positions of individual pieces of reception beam data with respect to the center of a transmission beam, and because, in the synthetic aperture imaging method, the sensitivity and the resolution differ among individual pieces of reception data upon synthesizing a plurality of pieces of reception data at each reception point, weighting processing is preferably applied in consideration of this. If invalidation processing is performed on data deviated from a valid area, the data processing efficiency can be enhanced.

In the above processing, it is possible to determine, for example, a point lower than a sound pressure peak by a sound pressure of predetermined dB (for example, -3dB) in the scanning direction as an edge of the transmission beam area. Further, in the above processing, it is also possible to form an ultrasonic image using the virtual source method without forming a reception beam. In that case, reception aperture processing is performed for each reception point in a predetermined order, thereby generating a pixel data array. In that case, the delay conditions are also set so that phases of the spherical waves match at the reception point.

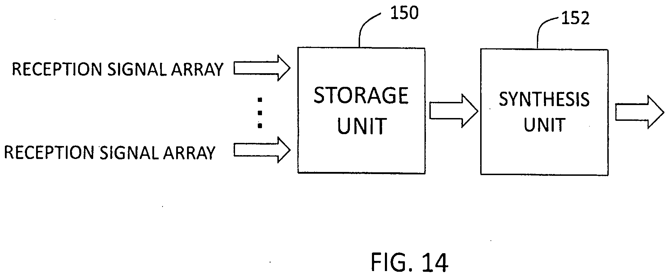

FIG. 14 shows a structure example of a reception unit in a second embodiment. A structure of a transmission unit is identical to that already described. Namely, a 2D virtual source array is formed on the scanning plane. A reception signal array (a plurality of element reception signals from a reception aperture) is obtained each time a virtual source is formed, and the arrays are sequentially stored in a storage unit 150. A plurality of reception signal arrays obtained by forming a plurality of virtual sources are stored in the storage unit 150. The plurality of reception signal arrays constitute a dataset. A synthesis unit 152 performs phasing addition processing using the dataset based on the virtual source method, thereby forming an ultrasonic image. In this second embodiment, the ultrasonic image is formed directly from the dataset without forming a plurality of sub-images. Because this second embodiment also forms an array where a plurality of virtual sources expand in two dimensions, it is possible to exclude or alleviate low sound pressure regions or low image quality regions generated between adjacent virtual sources on the scanning plane.

The above 2D virtual source array may be formed on each scanning plane formed in a 3D space. Further, 3D phasing addition processing may be realized by developing the above technique based on the virtual source method.

## Claims

1. An ultrasound diagnostic device comprising:
a transmission unit that forms a plurality of virtual sources as a plurality of transmission focal points on a scanning plane;
a sub-image forming unit that applies delay summing processing according to a virtual source method to a reception signal array obtained each time the virtual source is formed, thereby forming a sub-image; and
a synthesis unit that synthesizes a plurality of sub-images corresponding to the plurality of virtual sources, thereby generating an ultrasonic image, wherein
the plurality of virtual sources constitute a 2D virtual source array expanding in two dimensions on the scanning plane.

2. The ultrasound diagnostic device according to Claim 1, wherein
the virtual source array comprises a plurality of first virtual sources set at a first depth and a plurality of second virtual sources set at a second depth that differs from the first depth.

3. The ultrasound diagnostic device according to Claim 2, wherein
the plurality of first virtual sources and the plurality of second virtual sources are formed alternately in a scanning direction.

4. The ultrasound diagnostic device according to Claim 3, wherein
the transmission unit has
a function of transmitting ultrasonic waves from a first transmission aperture having a first aperture size, thereby forming a first transmission beam having a first transmission focal point set at the first depth, and
a function of transmitting ultrasonic waves from a second transmission aperture having a second aperture size that differs from the first aperture size, thereby forming a second transmission beam having a second transmission focal point set at the second depth,
the sub-image forming unit has
a function of forming a first reception beam data array based on a first reception signal array obtained by receiving reflected waves after the first transmission beam is formed, and
a function of forming a second reception beam data array based on a second reception signal array obtained by receiving reflected waves after the second transmission beam is formed,
a first sub-image is formed based on the first reception beam data, and
a second sub-image is formed based on the second reception beam data.

5. The ultrasound diagnostic device according to Claim 4, wherein
the first transmission beam has a first beam shape composed of a small inversed triangle area expanding from the first transmission focal point to the near side and a large triangle area expanding from the first transmission focal point to the far side, and
the second transmission beam has a second beam shape composed of a large inversed triangle area expanding from the second transmission focal point to the near side and a small triangle area expanding from the second transmission focal point to the far side.

6. The ultrasound diagnostic device according to Claim 1, wherein
the sub-image forming unit comprises
a delay summing processing unit that applies delay summing processing according to the virtual source method to a reception signal array obtained each time the virtual source is formed, thereby forming a reception beam data array, and
a weighting unit that applies a weight distribution to the reception beam data array.

7. The ultrasound diagnostic device according to Claim 6, wherein
the weighting unit provides a weight for invalidation to a portion of the reception beam data array, the portion deviated from a transmission beam area.

8. The ultrasound diagnostic device according to Claim 6, wherein
the transmission unit comprises
a function of transmitting ultrasonic waves from a first transmission aperture having a first aperture size, thereby forming a first transmission beam having a first transmission focal point set at the first depth, and
a function of transmitting ultrasonic waves from a second transmission aperture having a second aperture size that differs from the first aperture size, thereby forming a second transmission beam having a second transmission focal point set at a second depth that differs from the first depth,
the sub-image forming unit comprises
a function of forming a first reception beam data array based on a first reception signal array obtained by receiving reflected waves after the first transmission beam is formed, and
a function of forming a second reception beam data array based on a second reception signal array obtained by receiving reflected waves after the second transmission beam is formed, and
the weighting unit comprises
a function of applying a first weight distribution to the first reception beam data, thereby forming a first sub-image, and
a function of applying a second weight distribution to the second reception beam data, thereby forming a second sub-image.

9. The ultrasound diagnostic device according to Claim 1, wherein
the plurality of transmission focal points are formed by electronic linear scanning.

10. The ultrasound diagnostic device according to Claim 1, wherein
the plurality of transmission focal points are formed by electronic sector scanning.

11. An ultrasound diagnostic device comprising:
a transmission unit that forms a plurality of virtual sources as a plurality of transmission focal points on a scanning plane;
a storage unit that stores, as a dataset, a plurality of reception signal arrays obtained by forming the plurality of virtual sources; and
a synthesis unit that applies delay summing processing according to a virtual source method to the dataset, thereby generating an ultrasonic image, wherein
the plurality of virtual sources constitute a 2D virtual source array expanding in two dimensions on the scanning plane.

12. The ultrasound diagnostic device according to Claim 11, wherein
the virtual source array comprises a plurality of first virtual sources set at a first depth and a plurality of second virtual sources set at a second depth that differs from the first depth.

13. The ultrasound diagnostic device according to Claim 12, wherein
the plurality of first virtual sources and the plurality of second virtual sources are formed alternately in the scanning direction.
